# EUROPÄISCHE PATENTSCHRIFT

(11) **EP 0 946 630 B1**
(45) Veröffentlichungstag und Bekanntmachung des Hinweises auf die Patenterteilung: **09.05.2001**
(21) Anmeldenummer: 97953779.2
(22) Anmeldetag: 11.12.1997
(51) Int. Cl.: C08J 9/14, C09K 5/04

(54) **GEMISCHE MIT 1,1,1,3,3-PENTAFLUORBUTAN**
MIXTURES CONTAINING 1,1,1,3,3 PENTAFLUOROBUTANE
MELANGES CONTENANT DU 1,1,1,3,3-PENTAFLUOROBUTANE

(30) Priorität: 17.12.1996 DE 19652437; 16.06.1997 DE 19725360
(43) Veröffentlichungstag der Anmeldung: 06.10.1999
(73) Patentinhaber: Solvay Fluor und Derivate GmbH, 30173 Hannover (DE)
(72) Erfinder: KRÜCKE, Werner, D-30163 Hannover (DE); ZIPFEL, Lothar, D-30880 Laatzen (DE)
(74) Vertreter: Lauer, Dieter, Dr.
(86) Internationale Anmeldenummer: EP9706908
(87) Internationale Veröffentlichungsnummer: WO9827145

(56) Entgegenhaltungen:
- WO-A-96/12758

## Beschreibung

Die vorliegende Erfindung betrifft Gemische mit 1,1,1,3,3-Pentafluorbutan (R 365 mfc) und ihre Verwendung bei der Herstellung geschäumter Kunststoffe.

Es ist bereits bekannt, teilfluorierte Kohlenwasserstoffe als Treibgase für die Herstellung von geschäumten Kunststoffen zu verwenden. Die internationale Patentanmeldung WO 92/00345 offenbart die Verwendung teilweise fluorierter Alkane mit 4 oder 5 Kohlenstoffatomen, welcher eine "Tertiärstruktur" aufweisen, als Treibmittel für die Herstellung von Polyurethan- und Polyisocyanurat-Schaumkunststoffen.

Die deutsche Offenlegungsschrift DE 44 22 714 A1 offenbart Zusammensetzungen, die 1,1,2-Trifluorethan enthalten, und die Verwendung derartiger Zusammensetzungen als Treibmittel zur Herstellung geschäumter Kunststoffe. Die internationale Patentanmeldung WO 96/14354 offenbart die Herstellung geschäumter Kunststoffe, wobei eine flüssige Treibmittelzusammensetzung eingesetzt wird, die unter Druck verflüssigtes Kohlendioxid enthält. Die Treibmittelzusammensetzung kann darüber hinaus Fluorchlorkohlenwasserstoffe und Fluorkohlenwasserstoffe enthalten, beispielsweise auch 1,1,1,3,3-Pentafluorbutan (R 365 mfc). Die deutsche Patentanmeldung 195 41 013 offenbart u. a. Treibmittelzusammensetzungen zur Herstellung von Polyurethan-Weichschaumstoffen. Die Treibmittelzusammensetzungen umfassen 50 bis 95 Gewichtsteile 1,1,1,2-Tetrafluorethan und 5 bis 50 Gewichtsteile 1,1-Difluorethän und/oder leicht flüchtige organische Verbindungen. Die internationale Patentanmeldung WO 96/12758 offenbart Treibmittelmischungen für die Herstellung von PU-Hartschäumen. Die Treibmittel können gewünschtenfalls Fluorkohlenwasserstoffe wie 1,1,1,2-Tetrafluorethan; 1,1,1,3,3-Pentafluorpropan; 1,1,1,3,3-Pentafluorbutan oder 1,1,1,3,3,3-Hexafluorpropan enthalten. Die WO 96/30439 offenbart azeotrope Zusammensetzungen umfassend u. a. 1,1,1,4,4,4-Hexafluorbutan oder Perfluorhexan und bestimmte C5- oder C6-Kohlenwasserstoffe. Diese Zusammensetzungen können als Treibmittel für die Herstellung von Polyurethan-Hartschäumen verwendet werden.

Aufgabe der vorliegenden Erfindung ist es, ein Verfahren anzugeben, welches unter einfacherer Verarbeitung die Herstellung von vorteilhaften, geschäumten Kunststoffen, insbesondere Polyurethan-Schäumen, anzugeben. Aufgabe der vorliegenden Erfindung ist es weiterhin, Gemische ohne Flammpunkt anzugeben, mit welchen sich beispielsweise geschäumte Polymerkunststoffe mit qualitativ vorteilhaften Eigenschaften auf einfachere Weise erzeugen lassen.

Diese Aufgabe wird durch die vorliegende Erfindung gelöst.

Erfindungsgemäße, als Treibgas für die Herstellung geschäumter Kunststoffe brauchbare, Gemische enthalten oder bestehen aus 50 bis 99 Gew.-% 1,1,1,3,3-Pentafluorbutan (HFC 365 mfc) und 1 bis 50 Gew.-% mindestens eines Fluorkohlenwasserstoffes ausgewählt aus der Gruppe bestehend aus 1,1,1,2-Tetrafluorethan, 1,1,1,3,3-Pentafluorpropan, 1,1,1,3,3,3-Hexafluorpropan und 1,1,1,2,3,3,3-Heptafluorpropan.

1,1,1,3,3,-Pentafluorbutan liegt an der Grenze zur Entflammbarkeit. Die erfindungsgemäßen Gemische zeichnen sich beispielsweise dadurch aus, daß sie keine entflammbaren Flüssigkeiten bei 20 °C darstellen. Sie eignen sich besonders zur Anwendung als Treibgas für die Herstellung von geschäumten Kunststoffen, können aber auch für andere Zwecke wie beispielsweise als Kälte- , Lösungs- oder Reinigungsmittel eingesetzt werden.

Bevorzugte Gemische enthalten oder bestehen aus 80 bis 99 Gew.-% 1,1,1,3,3-Pentafluorbutan und 1 bis 20 Gew.-% 1,1,1,2-Tetrafluorethan, 1,1,1,3,3,3-Hexafluorpropan und/oder 1,1,1,2,3,3,3-Heptafluorpropan, insbesondere Gemische, die 80 bis 99 Gew.-% 365 mfc und 1 bis 20 Gew.-% 134a und/oder 227ea enthalten oder daraus bestehen.

Besonders bevorzugte Gemische mit 134a enthalten oder bestehen aus 91 bis 95 Gew.-% 1,1,1,3,3-Pentafluorbutan und 5 bis 9 Gew.-% 1,1,1,2-Tetrafluorethan; aus den angegebenen Verbindungen in den angegebenen Mengen bestehende Gemische weisen einen Siedepunkt von 20 °C auf.

Besonders bevorzugte Gemische mit 227ea sind solche, welche 80 bis 99 Gew.-% 365mfc und 1 bis 20 Gew.-% 227ea, insbesondere 85 bis 89 Gew.-% 1,1,1,3,3-Pentafluorbutan und 11 bis 15 Gew.-% 1,1,1,2,3,3,3-Heptafluorpropan enthalten oder daraus bestehen; aus den angegebenen Verbindungen in den zuletzt angegebenen Mengen bestehende Gemische weisen einen Siedepunkt im Bereich von ca. 23 °C auf.

Hervorragend sind auch Gemische mit 245fa, die 50 Gew.-% 1,1,1,3,3-Pentafluorbutan und 50 Gew.-% 1,1,1,3,3-Pentafluorpropan aufweisen mit einem Siedepunkt im Bereich von 22 °C. Diese Gemische ergeben bei der Anwendung besonders gute Dämmwerte.

Die erfindungsgemäßen Gemische liegen unter Standardbedingungen in flüssiger Form vor. Die bevorzugten Gemische sind z. B. bei Umgebungsdruck (ca. 1 atm) und 20 °C flüssig. In dieser Form werden sie zweckmäßig eingesetzt, wenn sie in zu verschäumende Kunststoffe oder deren Vormischungen eingearbeitet werden sollen. Sie eignen sich besonders gut als Treibgas für die Herstellung von geschäumten Kunststoffen, beispielsweise auch nach dem Extrusions-Verfahren. Bei diesem Verfahren werden die das Treibmittel enthaltenden Thermoplast-Kunststoffe direkt zu geschäumten Platten, Folien oder Profilen extrudiert. Die Kunststoffmasse schäumt unmittelbar nach Verlassen der Düse auf. Beispielsweise lassen sich XPS- und XPE-Schaumstoffe (Polystyrol- bzw. Polyethylen basierte Schaumstoffe) herstellen.

Besonders gut geeignet sind die erfindungsgemäßen Gemische zur Herstellung von Schaumstoffen auf Basis von Isocyanaten. Sie eignen sich sehr gut bei der Herstellung von insbesondere Hart-, aber auch Weichschäumen auf Isocyanat-Basis. Die Herstellung von Schaumstoffen auf Isocyanat-Basis ist bekannt. Ihre Herstellung und die dafür verwendbaren Grundmaterialien werden beispielsweise in der Europäischen Patentanmeldung EP-A-0 381 986; in "Ullmanns Encyclopedia of Industrial Chemistry", 5. Auflage, Band A 21, Seiten 665 bis 680; den internationalen Patentanmeldungen WO 92/00345, 96/30439, 96/14354 und der deutschen Offenlegungsschrift DE 44 22 714 A1 offenbart.

Als Ausgangskomponenten können aliphatische, cycloaliphatische, araliphatische, aromatische und heterocyclische Polyisocyanate beispielsweise mit 2 bis 4 Isocyanat-Gruppen eingesetzt werden. Sie weisen einen aliphatischen Kohlenwasserstoffrest mit bis zu 18 C-Atomen, einen cycloaliphatischen Kohlenwasserstoffrest mit bis zu 15 C-Atomen, einen aromatischen Kohlenwasserstoffrest mit 6 bis 15 C-Atomen oder einen araliphatischen Kohlenwasserstoffrest mit 8 bis 15 C-Atomen auf. Technisch besonders bevorzugte Ausgangskomponenten sind beispielsweise 2,4- und 2,6-Toluylendiisocyanat, Diphenylmethandiisocyanat, Polymethylenpolyphenylisocyanat und deren Mischungen. Es können auch sogenannte "modifizierte Polyisocyanate" eingesetzt werden, welche Carbodiimidgruppen, Urethangruppen, Allophanatgruppen, Isocyanuratgruppen, Harnstoffgruppen oder Biuretgruppen enthalten.

Weitere Ausgangskomponenten sind Verbindungen mit mindestens 2 gegenüber Isocyanaten reaktionsfähigen Wasserstoffatomen. Es handelt sich insbesondere um Verbindungen mit einem Molekurlargewicht von 400 bis 10.000, welche vorzugsweise 2 bis 8 Hydroxylgruppen aufweisen und außerdem Aminogruppen, Thiolgruppen oder Carboxylgruppen aufweisen können. Besonders gut geeignet sind Polyether, Polyester, Polycarbonate und Polyesteramide, die 2 bis 8 Hydroxylgruppen aufweisen.

Gegebenenfalls können auch Verbindungen als Ausgangskomponente eingesetzt werden, die als Kettenverlängerungsmittel oder oder Vernetzungsmittel dienen und vorzugsweise 2 bis 8 gegenüber Isocyanaten reaktionsfähige Wasserstoffatome aufweisen. Üblicherweise haben solche Mittel ein Molekular-gewicht von 32 bis 400. Anstelle oder zusätzlich zu Hydroxylgruppen können auch Aminogruppen, Thiolgruppen bzw. Carboxylgruppen vorhanden sein.

Gegebenenfalls können weitere Hilfs- und Zusatzmittel mitverwendet werden. Beispielsweise kann man zusätzlich chemische Treibmittel wie Wasser bzw. andere leicht flüchtige organische Substanzen als physikalisches Treibmittel einsetzen. Einsetzbar sind auch Katalysatoren wie beispielsweise tertiäre Amine, wie Dimethylcyclohexylamin, und/oder organische Metallverbindungen wie beispielsweise Zinnsalze von Carbonsäuren. Es können oberflächenaktive Zusatzstoffe wie Emulgatoren oder Schaumstabilisatoren, beispielsweise Siloxanpolyethercopolymere eingesetzt werden, Reaktionsverzögerer, Zellregler wie Parafine, Fettalkohole oder Dimethylpolysiloxane, Pigmente, Farbstoffe, Flammschutzmittel wie Phosphatester oder Phosphonatester, wie beispielsweise Trischloriso-propylphosphat. Einsetzbar sind weiterhin Stabilisatoren gegen Alterungs- und Witterungseinflüsse, Weichmacher, Füllstoffe, Farbstoffe, Antistatika, Nukleisierungsmittel, Porenreglersubstanzen oder biozid wirksame Wirkstoffe.

Gut geeignete Katalysatoren sind beispielsweise in der internationalen Patentanmeldung WO 96/14354 genannt. Dazu zählen organische Amine, Aminoalkohole und Aminoether wie Morpholinverbindungen, beispielsweise Dimethylcyclohexylamin, Diethanolamin, 2-Dimethylaminoethyl-3-dimethylaminopropyl-ether, 2-Dimethylaminoethylether, 2,2-Dimorpholinodiethyl-ether, N,N-Dimethylaminoethylmorpholin, N-Dimethylmorpholin. Auch metallorganische Verbindungen wie beispielsweise Zinn-, Kobalt- oder Eisenverbindungen sind brauchbar als Kataly-sator. Einsetzbar ist beispielsweise Zinndioxtoat, Kobalt-naphthenat, Dibutylzinndilaurat und Eisenacetonylacetat.

Ein weiterer Gegenstand der Erfindung sind Treibmittel zur Herstellung geschäumter Kunststoffe, die auf einem zur Verschäumung effektiven Gehalt des erfindungsgemäßen Gemisches als Treibgas basieren. Dabei können die Treibmittel aus dem erfindungsgemäßen Gemisch bestehen. Die Treibmittel können neben den erfindungsgemäßen Gemischen Hilfs- und Zusatzstoffe enthalten wie Wasser, einen oder mehrere Katalysatoren, Flammschutzmittel, Emulgatoren, Schaumstabilisatoren, Bindemittel, Vernetzungsmittel, UV-Stabilisatoren, Nukleirungsmittel und gegebenenfalls weitere Treibgase.

Ein weiterer Gegenstand der Erfindung sind bei Verwendung der erfindungsgemäßen Gemische erhaltene Kunststoffschäume, insbesondere solche, die in geschlossenen Zellen erfindungsgemäßes Gemisch enthalten. Bevorzugt sind entsprechende erfindungsgemäße Polyurethan-Hartschäume. Sie lassen sich aus verschäumbaren Mischungen herstellen, welche 1 bis 50 Gew.-% des erfindungsgemäßen Gemisches als Treibgas,bezogen auf Polyolkomponente, enthalten.

Auch Polyurethan-Weichschäume können unter Verwendung der erfindungsgemäßen Gemische als Treibgase hergestellt werden. Sie lassen sich aus an sich bekannten Rohstoff-Mischungen erzeugen, welche 1 bis 35 Gew.-% des erfindungsgemäßen Gemisches als Treibgas, bezogen auf die als 100 Gew.-% gesetzte Polyolkomponente, enthalten.

Die erfindungsgemäßen Gemische weisen den Vorteil auf, daß sie nicht brennbare Flüssigkeiten sind. Hingegen steht reines 1,1,1,3,3-Pentafluorbutan an der Schwelle zwischen brennbaren und nichtbrennbaren Substanzen. Außerdem hat es einen für manche Anwendungen zu hohen Siedepunkt. Die erfindungsgemäßen Gemische weisen den weiteren Vorteil auf, daß sie als Treibmittel für Polyurethan-Schäume Vorteile und eine einfachere Verarbeitung mit sich bringen können. Besonders günstig sind die angegebenen Gemische von R 365mfc mit R 134a im Gewichtsverhältnis 93:7 mit einem Siedepunkt von ca. 20 °C sowie Gemische von R 365mfc und R 227ea im Verhältnis 87:13 mit einem scheinbaren Siedepunkt von ca. 23 °C. Derartige Gemische entsprechen in ihrem Siedepunkt dem früher verwendeten R 11. Die Nichtbrennbarkeit der erfindungsgemäßen Gemische bedeutet für die Anwendung den Status eines Sicherheitstreibmittels wie er vormals für R 11 zugesprochen war. Durch Anpassung des effektiven Siedepunktes der Treibgase an die Rezeptur konnnte die Qualität der erhaltenen Schäume hinsichtlich des Dämmverhaltens verbessert werden, insbesondere bei der Anwendung für tiefe Temperaturen. So wird die Kondensation bei niedrigen Temperaturen verringert oder vermieden. Dementsprechend eignen sich die Schaumstoffe, insbesondere die Polyurethan-Schaumstoffe, die unter Verwendung eines erfindungsgemäßen Treibgases hergestellt worden sind, besonders gut für Dämmzwecke im Bau- und Wohnbereich, insbesondere auch deshalb, weil die in geschlossenen Zellen enthaltenen Zellgase eine hohe Wärmeisolierung mit sich bringen.

Die nachfolgenden Beispiele sollen die Erfindung näher erläutern, ohne sie jedoch in ihrem Umfang zu begrenzen.

### Beispiele

### Beispiel 1:

### Herstellung eines Gemisches von 1,1,1,3,3-Pentafluorbutan (R 365mfc) und 1,1,1,2-Tetrafluorethan (R 134a)

465 g R 365mfc wurde in einem Druckbehälter vorgelegt und 35 g R 134a dazugegeben. Es resultiert ein flüssiges Gemisch mit einem Siedepunkt von etwa 20 °C.

### Beispiel 2:

### Herstellung eines Gemisches von 1,1,1,3,3-Pentafluorbutan und 1,1,1,2,3,3,3-Heptafluorpropan (R 227ea)

435 g R 365 mfc wurde in einem Druckbehälter vorgelegt und 65 g R 227ea dazugegeben. Es resultiert ein flüssiges Gemisch mit einem Siedepunkt von etwa 23 °C.

### Beispiel 3:

### Herstellung eines Polyurethan-Hartschaumes mit R 365mf/R 134a als Treibgas

### Ansatz:

1.) Gemisch aus

| | |
|---|---|
| 100 g | Polyolmischung aus Polyetherpolyolen und einem aromatischen bromierten Polyetherpolyol, Hydroxylzahl 450 |
| 20 g | Tris-Chlorpropylphosphat (als Flammschutzmittel) |
| 2 g | Dimethylcyclohexylamin (als Katalysator) |
| 1,5 g | Siloxanpolyethercopolymer (als Schaumstabilisator) |
| 1 g | Wasser |
| 29 g | des gemäß Beispiel 1 hergestellten Gemisches (93 Teile 365mfc und 7 Teile R 134a) |

2.) 131 g 4,4'-Diisocyanatodiphenylmethan
Die unter 1.) genannten Stoffe wurden vermischt und das erhaltene Gemisch wurde mit dem Diisocyanat vermischt und zu einem PUR-Hartschaum verschäumt.
Der Schaumstoff hat eine Dichte von 35 kg/m³.

### Beispiel 4:

### Herstellung eines PUR-Hartschaumes mit R 365 mfc/R 227ea als Treibgas

Beispiel 3 wurde wiederholt. Als Treibgas wurden 30 g des gemäß Beispiel 2 hergestellten Gemisches eingesetzt.

Der erhaltene Schaumstoff entsprach in seiner Dichte dem gemäß Beispiel 3 erhaltenen Schaumstoff.

### Beispiel 5:

### Herstellung eines PUR-Hartschaumes mit R 365 mfc/R 134a als Treibgas unter Mitverwendung von Glycerin als Polyol

### Ansatz:

1.) Gemisch aus

| | |
|---|---|
| 100 g | Polyolkomponente aus einer Mischung von Polyetherpolyolen und einem aromatischen bromierten Polyetherpolyol, Hydroxylzahl 450 |
| 11 g | Dimethylmethylphosphonat |
| 1,8 g | einer Katalysatormischung |
| 2 g | Siloxanpolyethercopolymer |
| 1 g | Wasser |
| 32 g | des gemäß Beispiel 1 hergestellten Gemisches aus R 365mfc/R 134a |

2.) 170 g 4,4'-Diisocyanatodiphenylmethan
Das durch Vermischen der unter 1. genannten Komponenten erhaltene Gemisch wurde mit der Diisocyanat-Verbindung verschäumt. Bei der Verschäumung entstandt ein harter PUR-Schaum mit einer Rohdichte von 33 kg/m³.

### Beispiel 6:

### PUR-Hartschaum mit R 365mfc/R 227ea

Beispiel 5 wurde wiederholt. Jedoch wurden diesmal 37 g des in Beispiel 2 hergestellten Gemisches als physikalisches Treibgas eingesetzt. Die Dichte des Schaumes betrug 30 g/l.

### Beispiel 7:

### Herstellung eines Weichschaumes

### Ansatz:

1.) Gemisch aus

| | |
|---|---|
| 100 g | eines Polyetherpolyolen der Hydroxylzahl 56 |
| 0,05 g | Dibuthylzinndilaurat als Katalysator |
| 2 g | Siloxanpolyethercopolymer |
| 1 g | Wasser |
| 10 g | des gemäß Beispiel 1 hergestellten Gemisches aus R 365mfc/R 134a-Gemisches |

2.) 42 g Toluylendiisocyanat

Die Ausgangsstoffe wurden vermischt und verschäumt.

Der entstandene PUR-Weichschaum besaß eine Rohdichte von 22 kg/m³.

### Beispiel 8:

### Herstellung eines PUR-Weichschaumes

Beispiel 7 wurde wiederholt, jedoch unter Verwendung von 12 g des Gemisches gemäß Beispiel 2.

Die Rohdichte des Schaumes betrug 22 kg/m³.

## Patentansprüche

1. Gemisch enthaltend oder bestehend aus 50 bis 99 Gew.-% 1,1,1,3,3-Pentafluorbutan und 1 bis 50 Gew.-% mindestens eines Fluorkohlenwasserstoffes ausgewählt aus der Gruppe bestehend aus 1,1,1,2-Tetrafluorethan, 1,1,1,3,3-Pentafluorpropan, 1,1,1,3,3,3-Hexafluorpropan und 1,1,1,2,3,3,3-Heptafluorpropan.

2. Gemische nach Anspruch 1, enthaltend oder bestehend aus 80 bis 99 Gew.-% 1,1,1,3,3-Pentafluorbutan und 1 bis 20 Gew.-% 1,1,1,2-Tetrafluorethan, 1,1,1,3,3,3-Hexafluorpropan und/oder 1,1,1,2,3,3,3-Heptafluorpropan.

3. Gemisch nach Anspruch 2, enthaltend oder bestehend aus 91 bis 95 Gew.-% 1,1,1,3,3-Pentafluorbutan und 5 bis 9 Gew.-% 1,1,1,2-Tetrafluorethan mit einem Siedepunkt im Bereich von 20 °C.

4. Gemisch nach Anspruch 2, enthaltend oder bestehend aus 85 bis 89 Gew.-% 1,1,1,3,3-Pentafluorbutan und 11 bis 15 Gew.-% 1,1,1,2,3,3,3-Heptafluorpropan mit einem Siedepunkt im Bereich von 23 °C.

5. Gemische aus 50 Gew.-% 1,1,1,3,3-Pentafluorbutan und 50 Gew.-% 1,1,1,3,3-Pentafluorpropan mit einem Siedepunkt von 22 °C.

6. Gemische nach einem der vorhergehenden Ansprüche, dadurch gekennzeichnet, daß sie unter Standardbedingungen in flüssiger Form vorliegen.

7. Verwendung von Gemischen der Ansprüche 1 bis 6 als Treibgas für die Herstellung von geschäumten Kunststoffen.

8. Verwendung nach Anspruch 7, gekennzeichnet durch die Anwendung zur Herstellung von Polyurethan-Schaumstoffen und XPS/XPE-Schaumstoffen.

9. Verwendung nach Anspruch 8, gekennzeichnet durch die Anwendung zur Herstellung von Polyurethan-Hartschaumstoffen.

10. Verfahren zur Herstellung von geschäumten Kunststoffen unter Verwendung eines physikalischen Treibmittels auf Basis von Polyfluoralkan-Treibgasen, dadurch gekennzeichnet, daß man ein physikalisches Treibmittel mit einem Treibgas auf Basis der Gemische der Ansprüche 1 bis 6 einsetzt.

11. Treibmittel zur Herstellung geschäumter Kunststoffe, basierend auf einem zur Verschäumung effektiven Gehalt eines Gemisches der Ansprüche 1 bis 6 als Treibgas.

## Claims

1. A mixture containing or consisting of 50 to 99% by weight 1,1,1,3,3-pentafluorobutane and 1 to 50% by weight of at least one fluorohydrocarbon selected from the group consisting of 1,1,1,2-tetrafluoroethane, 1,1,1,3,3-pentafluoropropane, 1,1,1,3,3,3-hexafluoropropane and 1,1,1,2,3,3,3-heptafluoropropane.

2. Mixtures according to Claim 1, containing or consisting of 80 to 99% by weight 1,1,1,3,3-pentafluorobutane and 1 to 20% by weight 1,1,1,2-tetrafluoroethane, 1,1,1,3,3,3-hexafluoropropane and/or 1,1,1,2,3,3,3-heptafluoropropane.

3. A mixture according to Claim 2, containing or consisting of 91 to 95% by weight 1,1,1,3,3-pentafluorobutane and 5 to 9% by weight 1,1,1,2-tetrafluoroethane having a boiling point in the region of 20°C.

4. A mixture according to Claim 2, containing or consisting of 85 to 89% by weight 1,1,1,3,3-pentafluorobutane and 11 to 15% by weight 1,1,1,2,3,3,3-heptafluoropropane having a boiling point in the region of 23°C.

5. Mixtures of 50% by weight 1,1,1,3,3-pentafluorobutane and 50% by weight 1,1,1,3,3-pentafluoropropane having a boiling point of 22°C.

6. Mixtures according to one of the preceding claims, characterised in that they are in liquid form under standard conditions.

7. The use of mixtures according to Claims 1 to 6 as a blowing gas for the production of foamed plastics.

8. The use according to Claim 7, characterised by the application to the production of foamed polyurethane materials and XPS/XPE foamed materials.

9. The use according to Claim 8, characterised by the application to the production of rigid foamed polyurethane materials.

10. A process for the production of foamed plastics using a physical blowing agent based on polyfluoroalkane blowing gases, characterised in that a physical blowing agent with a blowing gas on the basis of the mixtures of Claims 1 to 6 is used.

11. A blowing agent for the production of foamed plastics, based on a content of a mixture of Claims 1 to 6, which is effective for foaming, as blowing gas.

## Revendications

1. Mélange contenant ou étant constitué de 50 à 99 % en poids de 1,1,1,3,3-pentafluorobutane et de 1 à 50 % en poids d'au moins un hydrocarbure fluoré choisi dans l'ensemble comprenant le 1,1,1,2-tétrafluoroéthane, le 1,1,1,3,3-pentafluoropropane, le 1,1,1,3,3,3-hexafluoropropane et le 1,1,1,2,3,3,3-heptafluoropropane.

2. Mélanges selon la revendication 1, contenant ou étant constitué de 80 à 99 % en poids de 1,1,1,3,3-pentafluorobutane et de 1 à 20 % en poids de 1,1,1,2-tétrafluoroéthane, de 1,1,1,3,3,3-hexafluoropropane et/ou de 1,1,1,2,3,3,3-heptafluoropropane.

3. Mélange selon la revendication 2, contenant ou étant constitué de 91 à 95 % en poids de 1,1,1,3,3-pentafluorobutane et de 5 à 9 % en poids de 1,1,1,2-tétrafluoroéthane, avec un point d'ébullition de l'ordre de 20°C.

4. Mélange selon la revendication 2, contenant ou étant constitué de 85 à 89 % en poids de 1,1,1,3,3-pentafluorobutane et de 11 à 15 % en poids de 1,1,1,2,3,3,3-heptafluoropropane, avec un point d'ébullition de l'ordre de 23°C.

5. Mélanges de 50 % en poids de 1,1,1,3,3-pentafluorobutane et de 50 % en poids de 1,1,1,3,3-pentafluoropropane, avec un point d'ébullition de 22°C.

6. Mélanges selon l'une des revendications précédentes, caractérisés en ce qu'ils se présentent sous forme liquide dans les conditions normales.

7. Utilisation de mélanges selon les revendications 1 à 6 en tant qu'agents porogènes pour fabriquer des plastiques expansés.

8. Utilisation selon la revendication 7, caractérisée par une utilisation pour fabriquer des mousses de polyuréthane et des mousses de XPS/XPE.

9. Utilisation selon la revendication 8, caractérisée par une utilisation pour fabriquer des mousses rigides de polyuréthane.

10. Procédé de fabrication de plastiques expansés par utilisation d'un agent porogène physique à base de gaz porogène de polyfluoralcane, caractérisé en ce qu'on utilise un agent porogène physique, avec un gaz porogène à base de mélanges selon les revendications 1 à 6.

11. Agent porogène pour fabriquer des plastiques expansés, à base d'une teneur, assurant une expansion, d'un mélange selon les revendications 1 à 6 en tant que gaz porogène.
